# EUROPÄISCHE PATENTSCHRIFT

(11) **EP 2 716 217 B1**
(45) Veröffentlichungstag und Bekanntmachung des Hinweises auf die Patenterteilung: **01.04.2015**
(21) Anmeldenummer: 13186675.8
(22) Anmeldetag: 30.09.2013
(51) Int. Cl.: A61B 5/15, A61B 5/151

(54) **Stechhilfevorrichtung zur Blutprobenentnahme**
Lancing device for taking blood samples
Dispositif d'aide à la piqûre pour le prélèvement d'échantillons de sang

(30) Priorität: 04.10.2012 DE 102012019400
(43) Veröffentlichungstag der Anmeldung: 09.04.2014
(73) Patentinhaber: Gerresheimer Regensburg GmbH, 93047 Regensburg (DE)
(72) Erfinder: Volkmuth, Julia, 93142 Maxhütte-Haidhof (DE); Eder, Andreas, 93051 Regensburg (DE)
(74) Vertreter: Hannke, Christian

(56) Entgegenhaltungen:
- US-A- 5 879 311
- US-A- 6 045 567
- US-A1- 2009 105 613
- US-A1- 2010 094 324

## Beschreibung

Die Erfindung betrifft eine Stechhilfevorrichtung zur Blutprobenentnahme mit einem Gehäuse, mit einem axial verlagerbaren Lanzettenhalterelement zum Haltern einer auswechselbaren Lanzette, mit einer Antriebseinheit umfassend wenigstens ein Stechfederelement zum Antreiben des axial verlagerbaren Lanzettenhalterelements in Stechrichtung und mit einem in einer Spannposition festlegbaren Spannschlittenteil zum Spannen des Stechfederelements.

Gattungsgemäße Stechhilfevorrichtungen mit einer Antriebseinheit aus insbesondere zwei in Reihe geschalteten Federelementen sind aus dem Stand der Technik bereits bekannt. Bei einer derartig ausgelegten Stechhilfevorrichtung sorgt ein vorderes Stechfederelement, also ein einem zu beschleunigenden Lanzettenhalter zugewandtes Federelement, für eine Beschleunigung einer durch diesen Lanzettenhalter gehalterten Lanzette während eines Stechvorgangs. Ein hinteres Rückholfederelement, also ein dem zu beschleunigenden Lanzettenhalter weiter abgewandtes Federelement, sorgt für eine Rückholbewegung des Lanzettenhalters nach dem Stechvorgang. Durch diese zwei derart miteinander wechselwirkenden Federelemente kann ein übermäßiges kritisches Nachschwingen des Lanzettenhalters an der Stechhilfevorrichtung nach einem Stechvorgang gut vermieden werden. Nachteilig bei diesen oder ähnlichen bekannten Stechhilfevorrichtungen ist ein oftmals konstruktiv aufwändiger Aufbau der Mechanik, der auch die Handhabung derartiger Stechhilfevorrichtungen erschwert.

Es ist Aufgabe der vorliegenden Erfindung, gattungsgemäße Stechhilfevorrichtungen hinsichtlich ihres konstruktiven Aufbaus und darüber hinaus hinsichtlich ihrer Handhabung zu vereinfachen.

Die Aufgabe der Erfindung wird von einer Stechhilfevorrichtung zur Blutprobenentnahme mit einem Gehäuse, mit einem axial verlagerbaren Lanzettenhalterelement zum Haltern einer auswechselbaren Lanzette, mit einer Antriebseinheit umfassend wenigstens ein Stechfederelement zum Antreiben des axial verlagerbaren Lanzettenhalterelements in Stechrichtung und mit einem in einer Spannposition festlegbaren Spannschlittenteil zum Spannen des Stechfederelements gelöst, wobei das festlegbare Spannschlittenteil in dieser Spannposition mittels einer an dem axial verlagerbaren Lanzettenhalterelement einrastbaren Rasteinrichtung temporär axial festgelegt ist.

Dadurch, dass das in der Spannposition festlegbare Spannschlittenteil in dieser Spannposition mittels der an dem axial verlagerbaren Lanzettenhalterelement einrastbaren Rasteinrichtung temporär axial festgelegt ist, vereinfacht sich der mechanische Aufbau der Stechhilfevorrichtung durch eine Bauteilreduzierung enorm.

Insbesondere wird hierdurch eine neue Auslösemöglichkeit hinsichtlich des Stechvorgangs erzielt, wodurch sich die Bedienung der vorliegenden Stechhilfevorrichtung wesentlich vereinfacht. Hierdurch wird ein verbesserter Umgang für einen Benutzer ermöglicht, durch welchen auch ein wesentlich verbessertes Stechergebnis erzielt wird.

Es versteht sich, dass die vorliegende Antriebseinheit vielfältig ausgestaltet sein kann. Vorteilhafterweise weist sie wenigstens zwei Federelemente umfassend ein Stechfederelement zum Beschleunigen des axial verlagerbaren Lanzettenhalterelements in die Stechrichtung und ein Rückholfederelement zum Beschleunigen des axial verlagerbaren Lanzettenhalterelements entgegen der Stechrichtung auf.

Insbesondere hierdurch ist ein Überschwingen der Federelemente vorteilhafterweise nicht erforderlich, da eine Vorwärtsbewegung während des Stechvorgangs und die sich daran anschließende Rückwärtsbewegung nach dem eigentlichen Stechvorgang durch zwei separate Federelemente realisiert werden.

Die hier verwendeten Federelemente umfassen vorzugsweise Schraubenfederelemente. Insofern handelt es sich bei dem in der Spannposition festlegbaren Spannschlittenteil vorzugsweise um einen Spannfederelementeschlittenteil.

Entlang der Stechrichtung bewegt sich vorliegend zumindest das axial verlagerbare Lanzettenhalterelement translatorisch, um einen Stechvorgang durchzuführen. Es bewegt sich entgegen dieser Stechrichtung translatorisch, um die Lanzette wieder zurück in das Gehäuse zu verlagern.

Konstruktiv vorteilhaft ist auch, dass das festlegbare Spannschlittenteil derart in dem Gehäuse gelagert ist, dass es sich ebenfalls entlang der Verlagerungsachse bewegen kann, um in die Spannposition hinein oder aus dieser heraus verlagert werden zu können.

Insofern formuliert diese Stechrichtung auch die Verlagerungsachse entlang welcher bzw. in deren Richtung sich insbesondere das axial verlagerbare Lanzettenhalterelement innerhalb des Gehäuses translatorisch bewegt.

Somit gestaltet idealerweise das axial verlagerbare Lanzettenhalterelement konstruktiv einfach ein Stechschlittenteil innerhalb des Gehäuses der vorliegenden Stechhilfevorrichtung aus.

Eine vorteilhafte Ausführungsvariante sieht vor, dass die Rasteinrichtung ein in Bezug zur Stechrichtung radial auslenkbares Rastarmelement umfasst, welches während des Festlegens des festlegbaren Spannschlittenteils in der Spannposition mit dem axial verlagerbaren Lanzettenhalterelement formschlüssig wechselwirkt. Hierdurch kann die einrastbare Rasteinrichtung baulich besonders einfach umgesetzt werden.

Ein derartiges auslenkbares Rastarmelement kann konstruktiv einfach idealerweise elastisch gebogen werden.

Vorzugsweise ist das elastisch auslenkbare Rastarmelement mindestens in zwei voneinander verschiedenen Wegen elastisch biegbar. Vorzugsweise verlaufen die zwei voneinander verschieden Wege hierbei quer zueinander.

Insofern ist das elastisch auslenkbare Rastarmelement mehrfach biegbar ausgestaltet.

Eine weitere vorteilhafte Ausführungsvariante sieht vor, dass die an dem axial verlagerbaren Lanzettenhalterelement einrastbare Rasteinrichtung Mittel zum Festlegen des festlegbaren Spannschlittenteils aufweist. Dies ist insbesondere dann vorteilhaft, wenn die einrastbare Rasteinrichtung nicht unmittelbar dem festlegbaren Spannschlittenteil zugeordnet ist.

Besonders vorteilhaft ist es in diesem Zusammenhang, wenn die einrastbare Rasteinrichtung ein in Bezug zur Stechrichtung radial auslenkbares Rastarmelement umfasst, welches Mittel zum insbesondere formschlüssigen Festlegen des festlegbaren Spannschlittenteils an dem Rastarmelement aufweist.

Die Mittel zum Festlegen des festlegbaren Spannschlittenteils können hierbei in vielfältiger Gestalt aufgeführt sein. Beispielsweise umfassen sie Federelemente oder Nutelemente einer Feder-Nut-Verbindung, welche mit den jeweils entsprechenden Gegenelementen am festlegbaren Spannschlittenteil wechselwirken können, wenn sich dieses in der Spannposition befindet. Hierdurch können insbesondere Mittel zum axialen Festlegen des festlegbaren Spannschlittenteils konstruktiv einfach realisiert werden.

Eine bevorzugte Ausführungsvariante sieht des Weiteren vor, dass die Rasteinrichtung derart in dem Gehäuse angeordnet ist, dass ein in Bezug zur Stechrichtung radial auslenkbares Rastarmelement durch eine translatorische Lageveränderung des axial verlagerbaren Lanzettenhalterelements in Stechrichtung radial ausrückbar angeordnet ist, so dass das festlegbare Spannschlittenteil unter Zuhilfenahme eines Druckfederelements translatorisch aus der Spannposition in eine Entspannposition verlagerbar ist. Hierdurch gelingt es auf baulich einfache Weise, das festlegbare Spannschlittenteil wieder in seine ursprüngliche Ruhelage bzw. Entspannposition zu überführen, um die Stechhilfevorrichtung betriebssicher für einen neuen Stechvorgang, beispielsweise für einen gefahrenlosen Lanzettenwechsel, vorzubereiten.

Darüber hinaus ist es vorteilhaft, wenn die Rasteinrichtung in Abhängigkeit von einer relativen Axiallage des axial verlagerbaren Lanzettenhalterelements gegenüber dem Gehäuse mit dem festlegbaren Spannschlittenteil wechselwirkt. Hierdurch kann ein sehr einfacher Aufbau der Mechanik erzielt werden.

Eine weitere konstruktive Vereinfachung kann erzielt werden, wenn die Rasteinrichtung ein Hülsenteil umfasst, welches das axial verlagerbare Lanzettenhalterelement außen zumindest teilweise radial umgibt.

Ein konstruktiv einfaches Wechselwirken zwischen der einrastbaren Rasteinrichtung und dem axial verlagerbaren Lanzettenhalterelement kann wesentlich verbessert werden, wenn das axial verlagerbare Lanzettenhalterelement eine von der einrastbaren Rasteinrichtung radial hintergreifbaren Rastrippe aufweist, welche an dem verlagerbaren Lanzettenhalterelement sich axial erstreckend angeordnet ist.

Die radial hintergreifbare Rastrippe ist vorteilhafterweise derart an dem axial verlagerbaren Lanzettenhalterelement platziert, dass sie von dem auslenkbaren Rastarmelement der einrastbaren Rasteinrichtung unerreichbar ist, wenn sich das axial verlagerbare Lanzettenhalterelement in seiner Stechendlage oder kurz davor befindet.

Insofern ist es vorteilhaft, wenn die Rasteinrichtung Mittel zum Verrasten mit dem axial verlagerbaren Lanzettenhalterelement aufweist. Beispielsweise umfassen die Mittel zum Verrasten wenigstens ein Rastnasenelement, welches die Rastrippe hintergreifen kann.

Insbesondere die Bedienung der Stechhilfevorrichtung kann darüber hinaus erheblich erleichtert werden, wenn eine das festlegbare Spannschlittenteil umfassende Spanneinheit ein weiteres Spannschlittenteil zum Verlagern des festlegbaren Spannschlittenteils umfasst, wobei das weitere Spannschlittenteil ein Einrückelement zum radialen Einrücken der an dem axial verlagerbaren Lanzettenhalterelement einrastbaren Rasteinrichtung aufweist.

Vorteilhafterweise ist dieses weitere Spannschlittenteil mittels eines außerhalb des Gehäuses zugänglichen Schiebers manuell entlang der Verlagerungsachse in Stechrichtung verschiebbar, so dass ein Verlagern des festlegbaren Spannschlittenteils in die Spannposition hinein händisch und konstruktiv einfach realisiert werden kann.

Hierbei ist der Schieber idealerweise in Längserstreckung der Stechhilfevorrichtung axial verschieblich an dem Gehäuse gelagert.

Auch das dem weiteren Spannschlittenteil zugeordnete Einrückelement kann konstruktiv vielfältig gestaltet sein. Hinsichtlich einer bevorzugten Konstruktionsvariante ist es vorteilhaft, wenn das Einrückelement eine gegenüber der Stechrichtung geneigte Kulissenführungsfläche umfasst. Hierdurch ist es baulich sehr platzsparend möglich, eine axial entlang der Verlagerungsachse gerichtete Bewegung des weiteren Spannschlittenteils in eine radial zur Verlagerungsachse gerichtete Bewegung der rastbaren Rasteinrichtung bzw. des auslenkbaren Rastarmelements umzulenken.

Die vorliegende Stechhilfevorrichtung kann allgemein noch kompakter und damit nochmals besser handhabbar gebaut werden, wenn das axial verlagerbare Lanzettenhalterelement, das festlegbare Spannschlittenteil oder das weitere Spannschlittenteil der Spanneinrichtung jeweils ein Hülsenteil umfassen.

Besonders vorteilhaft ist es hierbei, wenn einzelne der Hülsenteile derart in dem Gehäuse angeordnet sind, dass diese entlang der Verlagerungsachse beispielsweise ineinander geschoben werden können.

Insofern ist es außergewöhnlich vorteilhaft, wenn das axial verlagerbare Lanzettenhalterelement, das festlegbare Spannschlittenteil und/oder das weitere Spannschlittenteil eine gemeinsame Verlagerungsachse aufweisen.

Besonders vorteilhaft ist es auch, dass die vorliegende Kinematik der Antriebseinheit hinsichtlich des Spann- und des eigentlichen Stechvorgangs im Wesentlichen durch die zwei Spannschlittenteile der Spanneinheit und durch das Stechschlittenteil, welche zumindest teilweise ineinander verschieblich angeordnet sind, umgesetzt wird.

Eine weitere bauliche Vereinfachung der Stechhilfevorrichtung kann erzielt werden, wenn die Stechhilfevorrichtung ein zweiteiliges Gehäuse mit einem Oberschalenteil und einem Unterschalenteil aufweist, an welchen zumindest das weitere Spannschlittenteil direkt translatorisch geführt ist.

Insgesamt kann mit der erfindungsgemäßen Stechhilfevorrichtung eine außergewöhnlich gute ergonomische Handhabung einerseits aufgrund des einfachen Spannens des Stechfederelements durch Schieben des Schiebers erzielt werden.

Andererseits zeichnet sich die Stechhilfevorrichtung durch ein einfaches Auslösen des Lanzettenhalterelements bzw. des Stechschlittenteils durch radiales Drücken des Auslöseelements aus, da das axial verlagerbare Lanzettenhalterelement ein radial nach innen auslenkbares Fingerclipelement umfasst, welches sich im nicht ausgelösten Zustand der Stechhilfevorrichtung gegen einen radial nach innen ragenden Gehäusesteg abstützt, wodurch eine axiale Verlagerung des Lanzettenhalterelements in Stechrichtung unterbunden wird, solange das Auslöseelement noch nicht radial in das Gehäuse hineingedrückt wurde.

Insofern wird hierbei eine sequentielle Freigabe des axial verlagerbaren Lanzettenhalterelements und des fixierbaren Spannschlittenteils erzielt, welche nur eine einzige händische Aktion eines Benutzers erfordert, nämlich ein einstufiges Betätigen des Auslöseelements. Hierdurch vereinfacht sich die Handhabung vorliegender Stechhilfevorrichtung erheblich.

Da die vorliegende Rasteinrichtung und insbesondere das elastisch biegbare bzw. elastisch auslenkbare Rastarmelement hiervon nicht nur Mittel zum Festlegen des festlegbaren Spannschlittenteils umfasst sondern darüber hinaus vorteilhafterweise sogleich auch Mittel zum Verrasten, vorzugsweise in Gestalt eines Rastnasenelements, mit dem axial verlagerbaren Lanzettenhalterelement, kann die Mechanik der vorliegenden Stechhilfevorrichtung extrem einfach gestaltet werden.

Weitere Vorteile, Ziele und Eigenschaften vorliegender Erfindung werden anhand anliegender Zeichnung und nachfolgender Beschreibung erläutert, in welchen beispielhaft eine erfindungsgemäße Stechhilfevorrichtung zur Blutprobenentnahme mit einem axial verlagerbaren Lanzettenhalterelement und mit einem durch eine an dem axial verlagerbaren Lanzettenhalterelement einrastbare Rasteinrichtung temporär festlegbaren Spannschlittenteil dargestellt und beschrieben ist.

In der Zeichnung zeigen:
- Figur 1: schematisch eine Aufsicht einer Stechhilfevorrichtung zur Blutprobenentnahme mit einem durch eine an einem axial verlagerbaren Lanzettenhalterelement einrastbare Rasteinrichtung temporär festlegbaren Spannschlittenteil in einem Ruhezustand;
- Figur 2: schematisch eine Längsschnittseitenansicht der Stechhilfevorrichtung aus der Figur 1 in dem Ruhezustand;
- Figur 3: schematisch eine vordere Detailseitenansicht einer uneingerasteten Rasteinrichtung der Stechhilfevorrichtung gemäß der Figur 2;
- Figur 4: schematisch eine perspektivische Detailrückansicht der uneingerasteten Rasteinrichtung der Stechhilfevorrichtung aus den Figuren 1 bis 3;
- Figur 5: schematisch eine weitere Längsschnittseitenansicht der Stechhilfevorrichtung aus den Figuren 1 bis 4 in einem Spannzustand mit einer eingerasteten Rasteinrichtung, bei welchem sich ein weiteres Spannschlittenteil zum Axialverlagern des festlegbaren Spannschlittenteils in einer vorderen Schiebeposition befindet;
- Figur 6: schematisch eine weitere vordere Detailseitenansicht der eingerasteten Rastenrichtung der Stechhilfevorrichtung gemäß der Figur 5;
- Figur 7: schematisch eine nochmals weitere Längsschnittseitenansicht der Stechhilfevorrichtung aus den Figuren 1 bis 6 in dem Spannzustand, bei welchem sich das weiteres Spannschlittenteil zum Axialverlagern des festlegbaren Spannschlittenteils wieder in einer hinteren Ausgangsposition befindet;
- Figur 8: schematisch eine vordere Detailseitenansicht der eingerasteten Rasteinrichtung der Stechhilfevorrichtung gemäß der Figur 7;
- Figur 9: schematisch eine weitere perspektivische Detailrückansicht der eingerasteten Rasteinrichtung der Stechhilfevorrichtung aus den Figuren 1 bis 8 im Spannzustand;
- Figur 10: schematisch eine andere Längsschnittseitenansicht der Stechhilfevorrichtung aus den Figuren 1 bis 9 in einem ausgelösten Zustand; und
- Figur 11: schematisch eine weitere vordere Detailseitenansicht der Stechhilfevorrichtung gemäß der Figur 10.

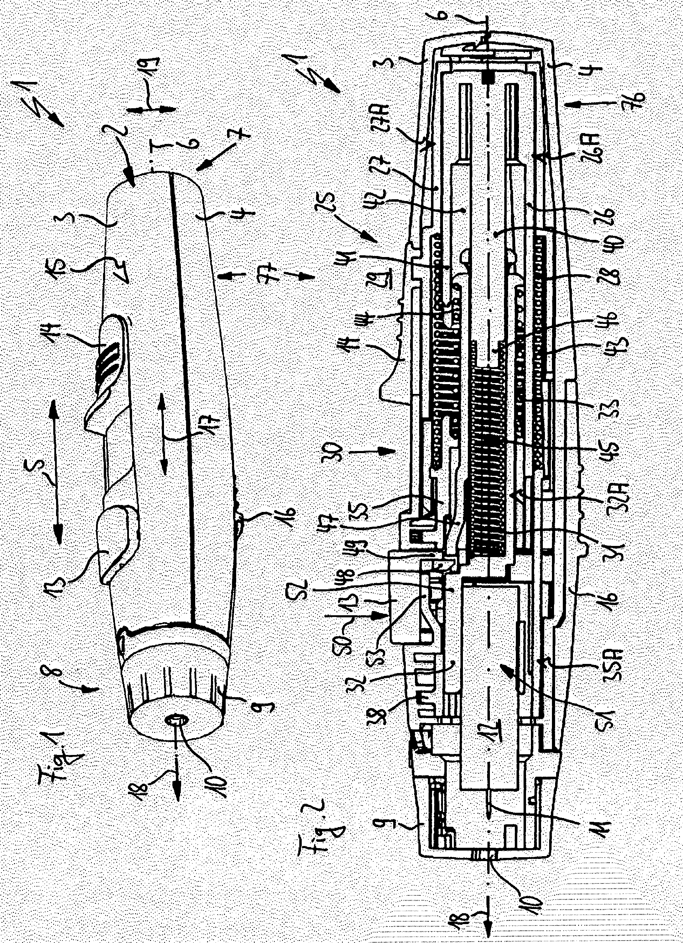

Die in den Figuren 1 bis 11 gezeigte Stechhilfevorrichtung 1 ist für eine Entnahme einer Blutprobe vorgesehen. Die Stechhilfevorrichtung 1 weist ein zweiteiliges Gehäuse 2 mit einem Oberschalenteil 3 und einem Unterschalenteil 4 auf.

Die Stechhilfevorrichtung 1 erstreckt sich mit einer Längserstreckung 5 entlang einer Längsachse 6 von einem Endbereich 7 der Stechhilfevorrichtung 1 zu einem Kopfbereich 8 der Stechhilfevorrichtung 1, wobei in diesem Kopfbereich 8 der Stechhilfevorrichtung 1 eine Schraubkappe 9 mit einer Durchführungsöffnung 10 für eine Stechnadel 11 einer auswechselbaren Lanzette 12 (siehe bspw. Figur 2) der Stechhilfevorrichtung 1 in das Gehäuse 2 eingeschraubt ist.

An dem Oberschalenteil 3 befinden sich radial neben der Längsachse 6 ein Auslösedrückelement 13 und ein Spannschiebeelement 14, welche beide über die Mantelfläche 15 des Gehäuses 2 radial hervorstehen, so dass sie händisch gut erreichbar sind.

An dem Unterschalenteil 4 befindet sich radial neben der Längsachse 6 ein Auswerferschiebeelement 16, welches ebenfalls über die Mantelfläche 15 des Gehäuses 2 radial hervorsteht, so dass auch dieses händisch problemlos erreichbar ist.

Während das Spannschiebeelement 14 und das Auswerferschiebeelement 16 in Axialrichtungen 17 und insbesondere in Stechrichtung 18 axial entlang der Längsachse 6 verlagerbar sind, ist das Auslösedrückelement 13 in Radialrichtungen 19 radial in Bezug auf die Längsachse 6 verlagerbar.

Das Spannschiebeelement 14 ist hierbei eine Komponente einer Spanneinheit 25 der Stechhilfevorrichtung 1, wobei die Spanneinheit 25 darüber hinaus zumindest ein festlegbares Spannschlittenteil 26 zum Spannen der Stechhilfevorrichtung 1, ein weiteres Spannschlittenteil 27 zum axialen Verlagern des festlegbaren Spannschlittenteils 26 sowie ein Spannschlittenfederelement 28 zum Rückverlagern des weiteren Spannschlittenteils 27 in eine hintere Ausgangsposition 29 nach einem Spannvorgang umfasst.

Das festlegbare Spannschlittenteil 26 und das weitere Spannschlittenteil 27 sind nicht fest miteinander fixiert, sondern axial relativ zueinander verschieblich gelagert; jedoch wird das festlegbare Spannschlittenteil 26 von dem weiteren Spannschlittenteil 27 mitgeschleppt, wenn das weitere Spannschlittenteil 27 in Stechrichtung 18 mithilfe des Spannschiebeelements 14 verlagert wird.

Sowohl das festlegbare Spannschlittenteil 26 als auch das weitere Spannschlittenteil 27 sind hierbei als Hülsenteile 26A und 27A ausgestaltet, wobei das Hülsenteil 26A des festlegbaren Spannschlittenteils 26 zumindest teilweise innerhalb des weiteren Spannschlittenteils 27 axial verschieblich gelagert angeordnet ist, und wobei das Hülsenteil 27A des weiteren Spannschlittenteils 27 in den Ober- und Unterschalenteilen 3 und 4 des zweigeteilten Gehäuses 2 axial verschieblich gelagert angeordnet ist.

Das weitere Spannschlittenteil 27 ist mit dem Spannschiebelement 14 formschlüssig verbunden (siehe insbesondere Figur 2), wodurch ersteres mit dem Spannschiebelement 14 händisch betätigt werden kann.

Des Weiteren verfügt die Stechhilfevorrichtung 1 über eine Antriebseinheit 30 umfassend zumindest ein Stechfederelement 31 zum Beschleunigen eines axial verlagerbaren Lanzettenhalterelements 32 in Stechrichtung 18 und ein Rückholfederelement 33 zum Beschleunigen des axial verlagerbaren Lanzettenhalterelements 32 entgegen die Stechrichtung 18 nach erfolgtem Stechvorgang.

Auch das axial verlagerbare Lanzettenhalterelement 32 ist vorliegend als ein Hülsenteil 32A ausgestaltet, in welchem einerseits das Hülsenteil 27A des festlegbaren Spannschlittenteils 27 beim Spannen der Stechhilfevorrichtung 1 zumindest teilweise axial eingeschoben werden kann.

Andererseits kann hierbei das Hülsenteil 32A des axial verlagerbaren Lanzettenhalterelements 32 zumindest teilweise in das Hülsenteil 27A des festlegbaren Spannschlittenteils 27 axial eingeschoben werden, wodurch eine sehr kompakt bauende und damit vorteilhaft handhabbare Stechhilfevorrichtung 1 realisiert werden kann.

Darüber hinaus umfasst die Stechhilfevorrichtung 1 noch eine Rasteinrichtung 35, welche als Hülsenteil 35A in dem zweiteiligen Gehäuse 2 an dem Ober- und Unterschalenteilen 3 und 4 fest und damit axial verschiebesicher fixiert ist.

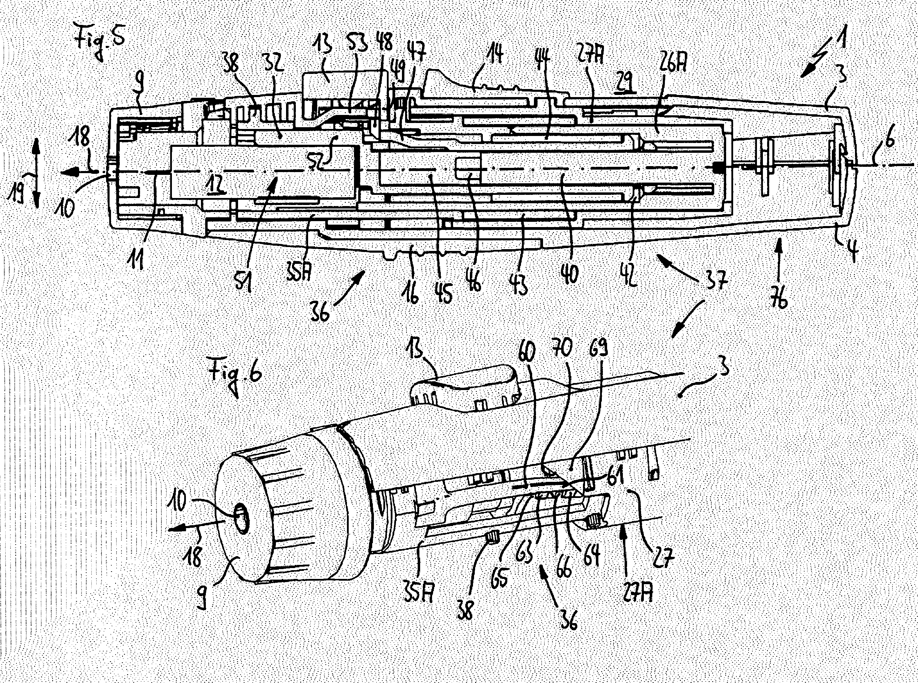

Mittels der Rasteinrichtung 35 kann das in Stechrichtung 18 geschobene festlegbare Spannschlittenteil 26 in einer Spannposition 36 (siehe insbesondere Figuren 5 bis 9) festgelegt werden, wie nachstehend auch noch detaillierter erläutert ist. Erfindungsgemäß ist die Rasteinrichtung 35 in einem solchen Spannzustand 37 der Stechhilfevorrichtung 1 an dem verlagerbaren Lanzettenhalterelement 32 derart eingerastet, dass bei einer axialen Verlagerung des Lanzettenhalterelements 32 in Stechrichtung 18 die Rasteinrichtung 35 ausrastet und damit das an der Rasteinrichtung festgelegte Spannschlittenteil 26 frei kommt und durch Federkraft des Stechfederelements 31 nach hinten in Richtung Endbereich 7 verlagert werden kann.

Vorteilhafterweise dient die Rasteinrichtung 35 gleichzeitig auch als Lager- und Führungseinrichtung zum axialen und radialen Lagern eines axial verlagerbaren Lanzettenhalterelements 32, wodurch die Stechhilfevorrichtung 1 nochmals bauteilreduzierter und damit kompakter baut.

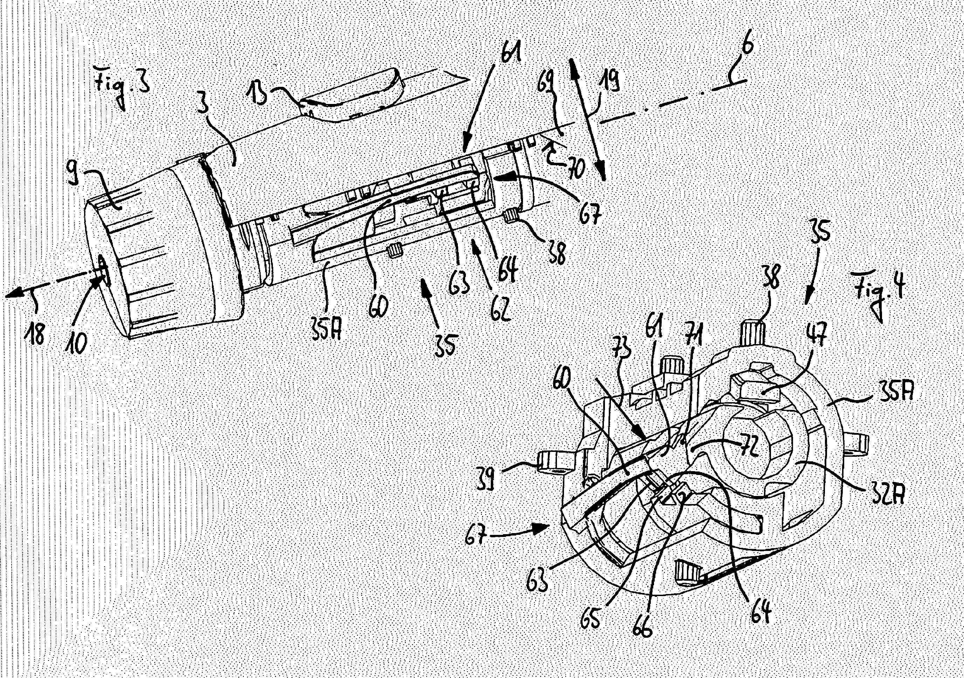

Um das Hülsenteil 35A der Rasteinrichtung 35 insbesondere verdrehsicher in dem Gehäuse 2 der Stechhilfevorrichtung 1 lagern zu können, weist das Hülsenteil 35A einige Stehbolzen 38 (hier nur exemplarisch beziffert) und Schraubenlaschen 39 (ebenfalls nur exemplarisch beziffert) auf (siehe beispielsweise Figuren 3 und 4), mithilfe welcher die Rasteinrichtung 35 an dem Gehäuse 2 ortsfest befestigt werden kann.

Aus der Darstellung gemäß der Figur 2 ist weiter gut zu erkennen, dass das festlegbare Spannschlittenteil 26 einen Innensteg 40, einen Außensteg 41 und einen dazwischenliegenden Zwischenraum 42 aufweist, in welchem das Hülsenteil 32A im Spannzustand 37 der Stechhilfevorrichtung 1 noch weiter hinein angeordnet werden kann.

Darüber hinaus bildet das Hülsenteil 35A der Rasteinrichtung 35, das Hülsenteil 26A des festlegbaren Spannschlittenteils 26 und das Hülsenteil 27A des weiteren Spannschlittenteils 27 einen Spannschlittenfederelementaufnahmeraum 43 aus, in welchem das Spannfederschlittenelement 28 gelagert ist.

Ebenso gestalten das Hülsenteil 35A der Rasteinrichtung 35 und das Hülsenteil 26A des festlegbaren Spannschlittenteils 26 im Zusammenwirken mit dem Hülsenteil 32A des axial verlagerbaren Lanzettenhalterelements 32 einen Rückholfederelementaufnahmeraum 44 aus.

Für das Stechfederelement 31 ist dementsprechend ein Stechfederelementaufnahmeraum 45 innerhalb der Stechhilfevorrichtung 1 vorgesehen, der vorteilhafterweise mithilfe des Hülsenteils 32A des axial verlagerbaren Lanzettenhalterelements 32 und dem Innensteg 40 des festlegbaren Spannschlittenteils 26 formuliert ist.

Eine verbesserte Führung des Stechfederelements 31 wird damit erzielt, dass der Innensteg 40 des festlegbaren Spannschlittenteils 26 mit einem durchmesserreduzierten Innenstegdorn 46 teilweise in das Stechfederelement 31 noch hineinragt.

Um das axial verlagerbare Lanzettenhalterelement 32 insbesondere während des Spannens der Stechhilfevorrichtung 1 und bei einem in der Spannposition 36 befindlichen festlegbaren Spannschlittenteil 26 gegen ein unbeabsichtigtes Vorschnellen in Stechrichtung 18 zu sichern, umfasst das axial verlagerbare Lanzettenhalterelement 32 ein radial elastisch biegbares Fingerclipelement 47, welches axial mit einem nach innen gerichteten Innenkragen 48 des Oberschalenteils 3 wechselwirken kann, wenn das axial verlagerbare Lanzettenhalterelement 32 durch Federkraft in Stechrichtung 18 verschoben wird.

Dieses radial elastisch biegbare Fingerclipelement 47 kann mithilfe eines Auslösedrückelementstegs 49 des Auslösedrückelements 13 in Betätigungsrichtung 50 radial nach innen gedrückt werden, wodurch es von dem Innenkragen 48 frei kommt und somit ein Stechvorgang in Stechrichtung 18 eingeleitet werden kann.

Im Bereich einer Lanzettenaufnahme 51, in welcher die auswechselbare Lanzette 12 an dem axial verlagerbaren Lanzettenhalterelement 32 gehaltert ist, besitzt das axial verlagerbare Lanzettenhalterelement 32 eine Außenwandung 52 , welche ebenfalls an dem nach innen gerichteten Innenkragen 48 des Oberschalenteils 3 anschlagen kann, wenn das axial verlagerbare Lanzettenhalterelement 32 zu weit entgegen die Stechrichtung 18 bewegt wird.

Zum radialen Zurückstellen des Auslösedrückelements 13 gestaltet das Oberschalenteil 3 noch ein federndes Rückstellelement 53 aus.

Alle Hülsenteile 26A, 27A, 32A und 35A liegen axial fluchtend in Längserstreckung 5 der Stechhilfevorrichtung 1 in dem Gehäuse, so dass sich ein kinematisch besonders einfacher und damit sehr betriebssicherer Aufbau und sich insofern eine einfach zu bedienende Stechhilfevorrichtung 1 ergeben.

Das Spannschlittenfederelement 28, das Stechfederelement 31 und das Rückholfederelement 33 sind hierbei idealerweise jeweils als Druckfederelemente in Gestalt von Schraubenfedern ausgeführt, wodurch sich der konstruktive Aufbau der vorliegenden Stechhilfevorrichtung 1 weiter vereinfachen lässt.

Nach den Darstellungen gemäß der Figuren 3 und 4 erkennt man sehr gut ein in Bezug zur axial verlaufenden Längsachse 6 und damit auch in Bezug zur Stechrichtung 18 radial elastisch auslenkbares Rastarmelement 60 der einrastbaren Rasteinrichtung 35, mithilfe dessen das festlegbare Spannschlittenteil 26 in der Spannposition 36 an der Rasteinrichtung 35 festgelegt werden kann.

Das elastisch auslenkbare Rastarmelement 60 verläuft hierbei in etwa parallel zur Längsachse 6 und es erstreckt sich axial entlang der Außenseite des axial verlagerbaren Lanzettenhalterelements 32.

Dieses elastisch auslenkbare Rastarmelement 60 weist an seinem freischwingenden Ende 61 Mittel 62 zum Festlegen des festlegbaren Spannschlittenteils 26 auf, welche in diesem Ausführungsbeispiel in Gestalt von Zahnelementen 63 und 64 ausgestaltet sind.

Diese Zahnelemente 63, 64 können mit an dem festlegbaren Spannschlittenteil 26 vorgesehenen Gegenzahnelementen 65 und 66 formschlüssig in Art einer Feder- Nut-Verzahnung wechselwirken (siehe Figuren 6, 8 und 9), wenn einerseits das festlegbare Spannschlittenteil 26 händisch in die Spannposition 36 axial nach vorne verschoben und andererseits das elastisch auslenkbare Rastarmelement 60 hierbei gleichzeitig aus seiner Freilage 67 (siehe Figuren 3 und 4) heraus in eine Sperrlage 68 hinein bewegt wurde.

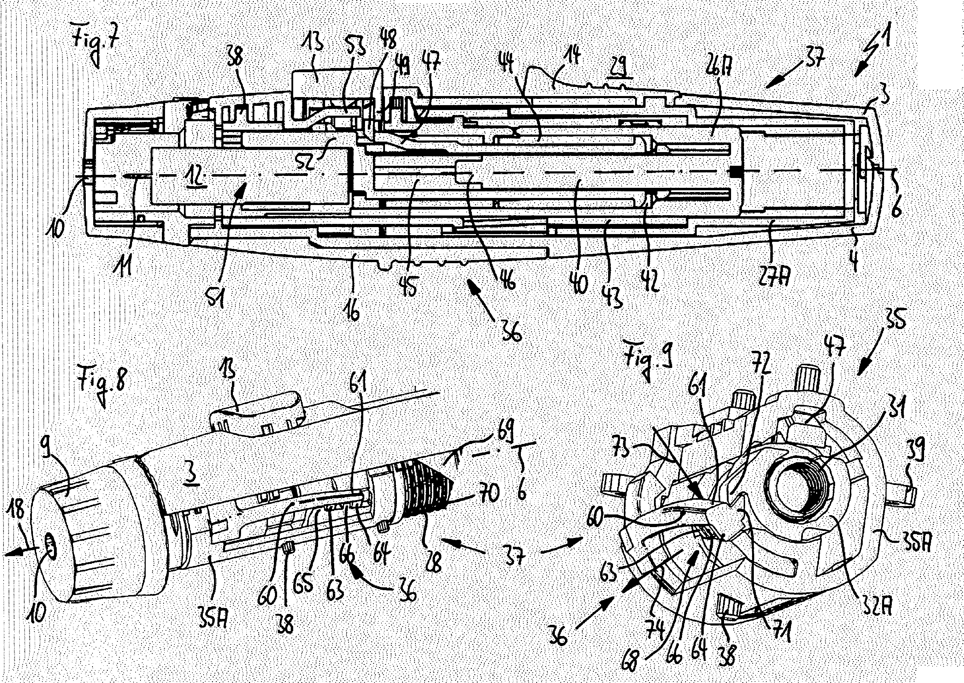

Um dieses Bewegen aus der Freilage 67 (siehe insbesondere Figur 4) heraus und in die Sperrlage 68 (siehe insbesondere Figur 9) hinein zu bewirken, weist das weitere Spannschlittenteil 27 ein Einrückelement 69 in Gestalt einer insbesondere hinsichtlich der Längsachse 6 geneigten Kulissenführungsfläche 70, welche entlang des elastisch auslenkbaren Rastarmelements 60 anliegend in Stechrichtung 18 vorbeigeführt werden kann. Hierbei wird das elastisch auslenkbare Rastarmelement 60 gebogen und entsprechend radial eingerückt.

Damit das elastisch auslenkbare Rastarmelement 60 in dieser Sperrlage 68 verbleiben kann, rastet es mit einem Rastnasenelement 71 formschlüssig hinter einer an dem axial verlagerbaren Lanzettenhalterelement 32 vorgesehenen Rastrippe 72 ein, indem das Rastnasenelement 71 die Rastrippe 72 hintergreift.

Hierzu ist das elastisch auslenkbare Rastarmelement 60 nicht nur in eine erste Auslenkrichtung 73 (siehe auch Figur 4) sondern vorteilhafterweise darüber hinaus ebenfalls auch noch in eine weitere Auslenkrichtung 74 (siehe Figur 9) auslenkbar, welche quer zu der ersten Auslenkrichtung 73 verläuft.

Diese radial hintergreifbare Rastrippe 72 erstreckt sich axial an dem axial verlagerbaren Lanzettenhalterelements 32 in Richtung der Längserstreckung 5.

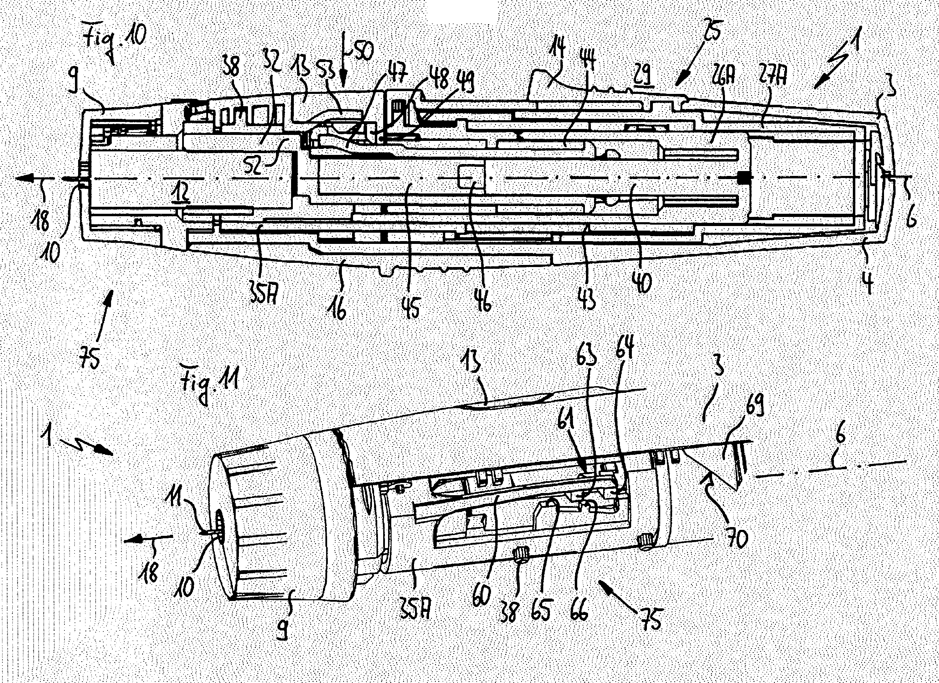

Wird nun in diesem gespannten Zustand der Stechhilfevorrichtung 1 das Auslösedrückelement 13 händisch betätigt, wird das Fingerclipelement 47 radial nach innen verlagert und kommt hierbei von dem Innenkragen 48 frei. Aufgrund des zuvor gespannten Stechfederelements 31 wird das axial verlagerbare Lanzettenhalterelement 32 nun in Stechrichtung 18 beschleunigt und erreicht letztendlich eine Stechendlage 75, wie dies in den Darstellungen gemäß der Figuren 10 und 11 gezeigt ist.

Spätestens in dieser Stechendlage 75 ist die hintergreifbare Rastrippe 72 mit dem axial verlagerbaren Lanzettenhalterelement 32 derart weit axial nach vorne in Stechrichtung 18 bewegt worden, dass das Rastnasenelement 71 nun frei axial hinter der hintergreifbaren Rastrippe 72 zurück bleibt, da dieses Rastnasenelement 71 axial ortsfest an dem elastisch auslenkbaren Rastarmelement 60 angeordnet ist.

Insofern überdecken sich das Rastnasenelement 71 und die durch dieses Rastnasenelement 71 hintergreifbare Rastrippe 72 nicht mehr, wenn das axial verlagerbare Lanzettenhalterelement 32 in Richtung der Stechendlage 75 unterwegs ist oder spätestens diese Stechendlage 75 erreicht.

Hierdurch kann sich das elastisch auslenkbare Rastarmelement 60 entgegen der ersten Auslenkrichtung 73 aus seiner Sperrlage 68 heraus und in seine Freilage 67 hinein bewegen, ohne dass das elastisch auslenkbare Rastarmelement 60 entgegen der weiteren Auslenkrichtung 74 bewegt werden muss.

Damit befinden sich dann die Zahnelemente 63 und 64 nicht mehr im Eingriff mit den Gegenzahnelementen 65 und 66, so dass das festlegbare Spannschlittenelement 26 aus seiner Spannposition 36 heraus in eine hintere Entspannposition 76 (siehe Figur 2) hinein beschleunigt wird.

Vorteilhafterweise ist die Rasteinrichtung somit derart in dem Gehäuse 2 angeordnet ist, dass das in Bezug zur Stechrichtung 18 radial elastisch auslenkbare Rastarmelement 60 durch eine translatorische Lageveränderung des axial verlagerbaren Lanzettenhalterelements 32 in die Stechrichtung 18 radial ausrückbar angeordnet ist, so dass das festlegbare Spannschlittenteil 26 unter Zuhilfenahme des Stechfederelements 31 translatorisch aus der Spannposition 36 in die Entspannposition 76 verlagert wird.

Die Stechhilfevorrichtung 1 befindet sich nun wieder in der in den Figuren 1 und 2 gezeigten Ruhelage 77 und kann für einen erneuten Stechvorgang präpariert werden.

Der vorstehend beschriebene Mechanismus kann auch angewendet werden, wenn das Spannschiebeelement 14 alternativ am Endbereich 7 als Druckknopf (hier nicht gezeigt) oder dergleichen platziert ist.

Es versteht sich, dass es sich bei dem vorstehend erläuterten Ausführungsbeispiel lediglich um erste Ausgestaltung der erfindungsgemäßen Stechhilfevorrichtung handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Stechhilfevorrichtung
- 2: zweiteiliges Gehäuse
- 3: Oberschalenteil
- 4: Unterschalenteil
- 5: Längserstreckung
- 6: Längsachse
- 7: Endbereich
- 8: Kopfbereich
- 9: Schraubkappe
- 10: Durchführungsöffnung
- 11: Stechnadel
- 12: Lanzette
- 13: Auslösedrückelement
- 14: Spannschiebeelement
- 15: Mantelfläche
- 16: Auswerferschiebeelement
- 17: Axialrichtungen
- 18: Stechrichtung
- 19: Radialrichtungen
- 25: Spanneinheit
- 26: festlegbares Spannschlittenteil
- 26A: Hülsenteil des festlegbaren Spannschlittenteils
- 27: weiteres Spannschlittenteil
- 27A: Hülsenteil des weiteren Spannschlittenteils
- 28: Spannschlittenfederelement
- 29: hintere Ausgangsposition
- 30: Antriebseinheit
- 31: Stechfederelement
- 32: axial verlagerbares Lanzettenhalterelement
- 32A: Hülsenteil des Lanzettenhalterelements
- 33: Rückholfederelement
- 35: Rasteinrichtung
- 35A: Hülsenteil der Rasteinrichtung
- 36: Spannposition
- 37: Spannzustand
- 38: Stehbolzen
- 39: Schraubenlaschen
- 40: Innensteg
- 41: Außensteg
- 42: Zwischenraum
- 43: Spannschlittenfederelementaufnahmeraum
- 44: Rückholfederelementaufnahmeraum
- 45: Stechfederelementaufnahmeraum
- 46: Innenstegdorn
- 47: Fingerclipelement
- 48: Innenkragen
- 49: Auslösedrückelementsteg
- 50: Betätigungsrichtung
- 51: Lanzettenaufnahme
- 52: Außenwandung
- 53: federndes Rückstellelement
- 60: elastisch auslenkbares Rastarmelement
- 61: freischwingendes Ende
- 62: Mittel zum Festlegen
- 63: vorderes Zahnelement
- 64: hinteres Zahnelement
- 65: vorderes Gegenzahnelement
- 66: hinteres Gegenzahnelement
- 67: Freilage
- 68: Sperrlage
- 69: Einrückelement
- 70: Kulissenführungsfläche
- 71: Rastnasenelement
- 72: radial hintergreifbare Rastrippe
- 73: erste Auslenkrichtung
- 74: weitere Auslenkrichtung
- 75: Stechendlage
- 76: Entspannposition
- 77: Ruhelage

## Patentansprüche

1. Stechhilfevorrichtung (1) zur Blutprobenentnahme mit einem Gehäuse (2), mit einem axial verlagerbaren Lanzettenhalterelement (32) zum Haltern einer auswechselbaren Lanzette (12), mit einer Antriebseinheit (30) umfassend wenigstens ein Stechfederelement (31) zum Antreiben des axial verlagerbaren Lanzettenhalterelements (32) in Stechrichtung (18) und mit einem in einer Spannposition (36) festlegbaren Spannschlittenteil (26) zum Spannen des Stechfederelements (31),
**dadurch gekennzeichnet, dass**
das festlegbare Spannschlittenteil (26) in dieser Spannposition (36) mittels einer an dem axial verlagerbaren Lanzettenhalterelement (32) einrastbaren Rasteinrichtung (35) temporär axial festgelegt ist.

2. Stechhilfevorrichtung (1) nach Anspruch 1,
**dadurch gekennzeichnet, dass**
die Rasteinrichtung (35) ein in Bezug zur Stechrichtung (18) radial auslenkbares Rastarmelement (60) umfasst, welches während des Festlegens des festlegbaren Spannschlittenteils (26) in der Spannposition (36) mit dem axial verlagerbaren Lanzettenhalterelement (32) formschlüssig wechselwirkt.

3. Stechhilfevorrichtung (1) nach Anspruch 1 oder 2,
**dadurch gekennzeichnet, dass**
das axial verlagerbare Lanzettenhalterelement (32) eine durch die Rasteinrichtung (35) radial hintergreifbaren Rastrippe (72) aufweist, welche an dem verlagerbaren Lanzettenhalterelement (32) sich axial erstreckend angeordnet ist.

4. Stechhilfevorrichtung (1) nach einem der Ansprüche 1 bis 3,
**dadurch gekennzeichnet, dass**
die einrastbare Rasteinrichtung (35) ein in Bezug zur Stechrichtung (18) radial auslenkbares Rastarmelement (60) umfasst, welches Mittel (62) zum Festlegen des festlegbaren Spannschlittenteils (26) an dem Rastarmelement (60) aufweist.

5. Stechhilfevorrichtung (1) nach einem der Ansprüche 1 bis 4,
**dadurch gekennzeichnet, dass**
die Rasteinrichtung (35) derart in dem Gehäuse (2) angeordnet ist, dass ein in Bezug zur Stechrichtung (18) radial auslenkbares Rastarmelement (60) durch eine translatorische Lageveränderung des axial verlagerbaren Lanzettenhalterelements (32) in Stechrichtung (18) radial ausrückbar angeordnet ist, so dass das festlegbare Spannschlittenteil (26) unter Zuhilfenahme eines Druckfederelements (31) translatorisch aus der Spannposition (36) in eine Entspannposition (76) verlagerbar ist.

6. Stechhilfevorrichtung (1) nach einem der Ansprüche 1 bis 5,
**dadurch gekennzeichnet, dass**
die Rasteinrichtung (35) in Abhängigkeit von einer relativen Axiallage des axial verlagerbaren Lanzettenhalterelements (32) gegenüber dem Gehäuse (2) mit dem festlegbaren Spannschlittenteil (26) wechselwirkt.

7. Stechhilfevorrichtung (1) nach einem der Ansprüche 1 bis 6,
**dadurch gekennzeichnet, dass**
die Rasteinrichtung (35) ein Hülsenteil (35A) umfasst, welches das axial verlagerbare Lanzettenhalterelement (32) außen zumindest teilweise radial umgibt.

8. Stechhilfevorrichtung (1) nach einem der Ansprüche 1 bis 7,
**dadurch gekennzeichnet, dass**
die Spanneinheit (25) ein weiteres Spannschlittenteil (27) zum Verlagern des festlegbaren Spannschlittenteils (26) umfasst, wobei das weitere Spannschlittenteil (27) ein Einrückelement (69) zum radialen Einrücken der an dem axial verlagerbaren Lanzettenhalterelement (32) einrastbaren Rasteinrichtung (35) aufweist.

9. Stechhilfevorrichtung (1) nach Anspruch 8,
**dadurch gekennzeichnet, dass**
das Einrückelement (69) eine gegenüber der Stechrichtung (18) geneigte Kulissenführungsfläche (70) umfasst.

10. Stechhilfevorrichtung (1) nach einem der Ansprüche 1 bis 9
**dadurch gekennzeichnet, dass**
das axial verlagerbare Lanzettenhalterelement (32), das festlegbare Spannschlittenteil (26) oder ein weiteres Spannschlittenteil (27) jeweils ein Hülsenteil (32A, 26A, 27A) umfassen.

## Claims

1. Lancing device (1) for taking blood samples, with a housing (2), an axially displaceable lancet holder element (32) for holding a replaceable lancet (12), a drive unit (30) comprising at least one lancing spring element (31) for driving the axially displaceable lancet holder element (32) in the lancing direction (18), and a tensioning carriage part (26) which can be fixed in a tensioning position (36) for tensioning the lancing spring element (31), **characterised in that** the fixable tensioning carriage part (26) is temporarily axially fixed in said tensioning position (36) by means of a latching apparatus (35) which can be latched to the axially displaceable lancet holder element (32).

2. Lancing device (1) according to claim 1, **characterised in that** the latching apparatus (35) comprises a latching arm element (60) which can be deflected radially in relation to the lancing direction (18), which interacts in an interlocking manner with the axially displaceable lancet holder element (32) during fixing of the fixable tensioning carriage part (26) in the tensioning position (36).

3. Lancing device (1) according to either claim 1 or claim 2, **characterised in that** the axially displaceable lancet holder element (32) comprises a latching rib (72) which can be radially engaged from behind by the latching apparatus (35), which is arranged such that it extends axially on the displaceable lancet holder element (32).

4. Lancing device (1) according to any of claims 1 to 3, **characterised in that** the latchable latching apparatus (35) comprises a latching arm element (60) which can be deflected radially in relation to the lancing direction (18), which comprises means (62) for fixing the fixable tensioning carriage part (26) to the latching arm element (60).

5. Lancing device (1) according to any of claims 1 to 4, **characterised in that** the latching apparatus (35) is arranged in the housing (2) such that a latching arm element (60) which can be deflected radially in relation to the lancing direction (18) is arranged such that it can be disengaged radially by a translational change in position of the axially displaceable lancet holder element (32) in the lancing direction (18), such that the fixable tensioning carriage part (26) can be translationally displaced out of the tensioning position (36) into a relaxed position (76) with the aid of a compression spring element (31).

6. Lancing device (1) according to any of claims 1 to 5, **characterised in that** the latching apparatus (35) interacts with the fixable tensioning carriage part (26) depending on a relative axial position of the axially displaceable lancet holder element (32) in relation to the housing (2).

7. Lancing device (1) according to any of claims 1 to 6, **characterised in that** the latching apparatus (35) comprises a sleeve part (35A) which radially surrounds the outside of the axially displaceable lancet holder element (32) at least in part.

8. Lancing device (1) according to any of claims 1 to 7, **characterised in that** the tensioning unit (25) comprises a further tensioning carriage part (27) for displacing the fixable tensioning carriage part (26), the further tensioning carriage part (27) comprising an engagement element (69) for radially engaging the latching apparatus (35) which can be latched to the axially displaceable lancet holder element (32).

9. Lancing device (1) according to claim 8, **characterised in that** the engagement element (69) comprises a connecting link guide surface (70) which is inclined in relation to the lancing direction (18).

10. Lancing device (1) according to any of claims 1 to 9, **characterised in that** the axially displaceable lancet holder element (32), the fixable tensioning carriage part (26) or a further tensioning carriage part (27) each comprise a sleeve part (32A, 26A, 27A).

## Revendications

1. Dispositif d'aide à la piqûre (1) pour le prélèvement d'échantillons de sang, avec un boîtier (2), avec un élément de support de lancette (32) mobile axialement pour supporter une lancette (12) interchangeable, avec une unité d'entraînement (30) comprenant au moins un élément de ressort de piqûre (31) pour l'entraînement, dans la direction de piqûre (18), de l'élément de support de lancette (32) mobile axialement, et avec une partie de coulisseau de serrage (26) pouvant être immobilisée dans une position de serrage (36) pour le serrage de l'élément de ressort de piqûre (31),
**caractérisé en ce que**
la partie de coulisseau de serrage (26) pouvant être immobilisée est immobilisée axialement provisoirement dans cette position de serrage (36) au moyen d'un dispositif d'encliquetage (35) pouvant être encliqueté sur l'élément de support de lancette (32) mobile axialement.

2. Dispositif d'aide à la piqûre (1) selon la revendication 1,
**caractérisé en ce que**
le dispositif d'encliquetage (35) comprend un élément de bras d'encliquetage (60) pouvant être dévié radialement par rapport à la direction de piqûre (18) et qui, pendant l'immobilisation de la partie de coulisseau de serrage (26) pouvant être immobilisée dans la position de serrage (36), interagit par liaison de forme avec l'élément de support de lancette (32) mobile axialement.

3. Dispositif d'aide à la piqûre (1) selon la revendication 1 ou 2,
**caractérisé en ce que**
l'élément de support de lancette (32) mobile axialement présente une nervure d'encliquetage (72) pouvant être agrippée par l'arrière radialement par le dispositif d'encliquetage (35), qui est disposée en s'étendant axialement sur l'élément de support de lancette (32) mobile.

4. Dispositif d'aide à la piqûre (1) selon une des revendications 1 à 3,
**caractérisé en ce que**
le dispositif d'encliquetage (35) encliquetable comprend un élément de bras d'encliquetage (60) pouvant être dévié radialement par rapport à la direction de piqûre (18), qui présente des moyens (62) pour l'immobilisation de la partie de coulisseau de serrage (26) pouvant être immobilisée sur l'élément de bras d'encliquetage (60).

5. Dispositif d'aide à la piqûre (1) selon une des revendications 1 à 4, **caractérisé en ce que**
le dispositif d'encliquetage (35) est disposé dans le boîtier (2) de telle sorte qu'un élément de bras d'encliquetage (60) pouvant être dévié radialement par rapport à la direction de piqûre (18) est disposé de façon à pouvoir être libéré radialement dans la direction de piqûre (18) par une modification de position en translation de l'élément de support de lancette (32) mobile axialement de telle sorte que la partie de coulisseau de serrage (26) pouvant être immobilisée peut, avec l'aide d'un élément à ressort de compression (31), être déplacée en translation à partir de la position de serrage (36) vers une position de desserrage (76).

6. Dispositif d'aide à la piqûre (1) selon une des revendications 1 à 5, **caractérisé en ce que**
le dispositif d'encliquetage (35) interagit avec la partie de coulisseau de serrage (26) pouvant être immobilisée en fonction d'une position axiale relative de l'élément de support de lancette (32) mobile axialement par rapport au boîtier (2).

7. Dispositif d'aide à la piqûre (1) selon une des revendications 1 à 6, **caractérisé en ce que**
le dispositif d'encliquetage (35) comprend une partie de douille (35A) qui entoure extérieurement au moins de façon partiellement radiale l'élément de support de lancette (32) mobile axialement.

8. Dispositif d'aide à la piqûre (1) selon une des revendications 1 à 7, **caractérisé en ce que**
l'unité de serrage (25) comprend une autre partie de coulisseau de serrage (27) pour le déplacement de la partie de coulisseau de serrage (26) pouvant être immobilisée, l'autre partie de coulisseau de serrage (27) présentant un élément d'engagement (69) pour l'engagement radial du dispositif d'encliquetage (35) encliquetable sur l'élément de support de lancette (32) mobile axialement.

9. Dispositif d'aide à la piqûre (1) selon la revendication 8,
**caractérisé en ce que**
l'élément d'engagement (69) comprend une surface de guidage à coulisse (70) inclinée par rapport à la direction de piqûre (18).

10. Dispositif d'aide à la piqûre (1) selon une des revendications 1 à 9, **caractérisé en ce que**
l'élément de support de lancette (32) mobile axialement, la partie de coulisseau de serrage (26) pouvant être immobilisée ou un autre partie de coulisseau de serrage (27) comprennent respectivement une partie de douille (32A, 26A, 27A).
